# EUROPEAN PATENT APPLICATION

(11) **EP 3 974 060 A1**
(43) Date of publication of application: **30.03.2022**
(21) Application number: 21153634.7
(22) Date of filing: 27.01.2021
(51) Int. Cl.: B01L 3/00

(54) **MICROFLUIDIC CHIP AND DEVICE**

(30) Priority: 24.09.2020 US 202017030400
(71) Applicant: MiCareo Taiwan Co., Ltd., 114 Taipei City (TW)
(72) Inventor: Yu, Hui-Min, 114 Taipei City (TW); Fang, Wei-Feng, 114 Taipei City (TW); Chang, Ching-Chih, 114 Taipei City (TW); Chen, Jui-Lin, 114 Taipei City (TW)
(74) Representative: Becker, Eberhard

(57) **Abstract**

A microfluidic device and a microfluidic chip are provided. The microfluidic device includes the microfluidic chip, a pouring element, a flow adjustment element and a processor. The microfluidic chip includes a sorting assembly, a sample outlet channel, a pouring channel, a collection channel and a waste channel. The sorting assembly includes a sample inlet channel and a sorting chamber. The pouring element is connected to the pouring channel. The flow adjustment element is connected to a distal end of the sample outlet channel. The processor is configured to control the pouring element to pour a guiding fluid into the pouring channel entering the sample outlet channel and control the flow adjustment element to adjust a flow resistance of a drain section of the sample outlet channel.

## Description

### BACKGROUND

### Technical Field

The present disclosure is related to chips and devices for collecting target particles/cells in a fluidic sample, and more particularly related to microfluidic chips and devices.

### Description of Related Art

Microfluidic chips and devices have been widely applied in various fields, particularly in the bio-related fields, such as the biomedical field, the biochemical field and so on. In the application of the bio-related field, a blood sample containing various cells is loaded in a microfluidic chip for collecting at least one target cell among the various cells. The ability to collect the target cells via the microfluidic chip or device represents significant advance in disease screening and monitoring.

### SUMMARY

The present application provides a microfluidic chip and device for accurately sorting and collecting at least one target cell in a fluidic sample.

In accordance with some embodiments of the present application, a microfluidic device and a microfluidic chip are provided, wherein the microfluidic device includes the microfluidic chip, and further includes a pouring element, a flow adjustment element and a processor. The microfluidic chip includes a first sorting assembly, a first sample outlet channel, a pouring channel, a collection channel and a first waste channel. The first sorting assembly includes a first sorting chamber, a first sample inlet channel, and two guiding channels, wherein the first sample inlet channel and the two guiding channels are converged at the first side of the first sorting chamber. The first sample inlet channel is positioned between the two guiding channels. The first sample inlet channel extends to reach a first side of the first sorting chamber and is configured to allow a fluidic sample entering the first sorting chamber. The first sample outlet channel extends to reach a second side of the first sorting chamber at an inlet end of the first sample outlet channel. The pouring channel is branched from the first sample outlet channel at a first joint of the first sample outlet channel. The collection channel is branched from the first sample outlet channel at a second joint of the first sample outlet channel and includes an ejection hole connected to a droplet ejection device to dispense a single droplet. The first sorting chamber bifurcates into the first waste channel and the first sample outlet channel at the second side. The first sample outlet channel consists of an entrance section positioned between the inlet end of the fist sample outlet and the first joint, a buffer section positioned between the first joint and the second joint, and a drain section positioned between the second joint and the distal end of the first sample outlet. The pouring element is connected to the pouring channel. The flow adjustment element is connected to a distal end of the first sample outlet channel. When the flow adjustment element is actuated, a flow resistance to the fluid flow in the drain section is lower than a flow resistance to the fluid flow in the collection channel. The processor is configured to control the pouring element to pour a guiding fluid into the pouring channel entering the buffer section of the first sample outlet channel and control the flow adjustment element to adjust a flow resistance of the drain section of the first sample outlet channel.

To make the aforementioned more comprehensible, several embodiments accompanied with drawings are described in detail as follows.

### BRIEF DESCRIPTION OF THE DRAWINGS

The accompanying drawings are included to provide a further understanding of the disclosure, and are incorporated in and constitute a part of this specification. The drawings illustrate exemplary embodiments of the disclosure and, together with the description, serve to explain the principles of the disclosure.
FIG. 1 depicts a microfluidic device according to an embodiment of the present application.
FIGS. 2A-2C depict an enlarged partial view of the microfluidic device of FIG. 1.
FIG. 3 depicts a microfluidic chip according to an embodiment of the present application.
FIG. 4 depicts a microfluidic chip according to an embodiment of the present application.

### DESCRIPTION OF THE EMBODIMENTS

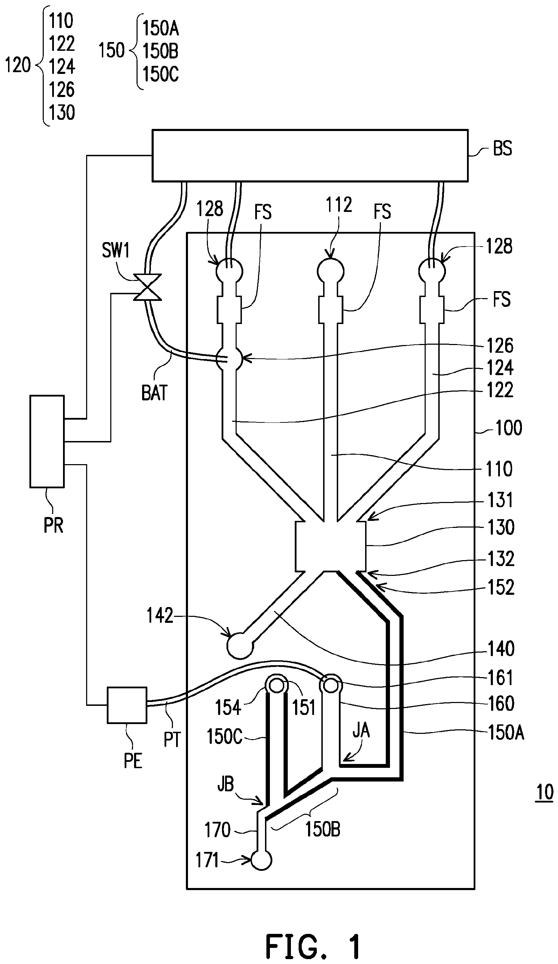

Refer to FIG. 1, which depicts a microfluidic device for sorting and collecting at least one target cell in a fluidic sample according to an embodiment of the present application. The microfluidic device 10 includes the microfluidic chip 100. The microfluidic chip 100 includes a first sorting assembly 120, a first sample outlet channel 150, a pouring channel 160, a collection channel 170 and a first waste channel 140. The first sorting assembly 120 includes a first sample inlet channel 110. Specifically, a fluidic sample to-be processed may be injected into the microfluidic chip 100 through the first sample inlet channel 110 and later sorted by the first sorting assembly 120. When no target cell is detected in the fluidic sample, the fluidic sample is drained out through the first waste channel 140. Alternately, when at least one target cell is detected in the fluidic sample, the fluidic sample with the target cell is sorted by the sorting operation of the first sorting assembly 120 and pushed to enter the first sample outlet channel 150. The target cell entering the first sample outlet channel 150 may be ejected from the collection channel 170 by pouring a fluid flow from the pouring channel 160 into the first sample outlet channel 150 to push the target cell to travel towards the collection channel 170.

The first sorting assembly 120 further includes two first guiding channels 122 and 124 and a first sorting chamber 130. The first sample inlet channel 110 extends to reach a first side 131 of the first sorting chamber 130 and includes a fluidic sample inlet hole 112. In other words, the first sample inlet channel 110 extends between the first side 131 of the first sorting chamber 130 and the fluidic sample inlet hole 112. The first sample inlet channel 110 and the fluidic sample inlet hole 112 are configured to allow a fluidic sample entering the first sorting chamber 130. The first sample inlet channel 110 and the two first guiding channels 122 and 124 are converged at the first side 131 of the first sorting chamber 130. The joint of the first sample inlet channel 110 connecting to the first side 131 of the first sorting chamber 130 is positioned between joints of the two first guiding channels 122 and 124 connecting to the first side 131 of the first sorting chamber 130, as shown in FIG. 1.

The first sorting chamber 130 bifurcates into the first waste channel 140 and the first sample outlet channel 150 at a second side 132 of the first sorting chamber 130. The first waste channel 140 extends between the second side 132 of the first sorting chamber 130 and a waste outlet hole 142 passing through the outer surface of the microfluidic chip 100 and forming a fluidic communication with the first waste channel 140. The first sample outlet channel 150 may have an inlet end 152 reaching a second side 132 of the first sorting chamber 130 and a distal end 154 distant from the inlet end 152. The pouring channel 160 is branched from the first sample outlet channel 150 at a first joint JA of the first sample outlet channel 150. The collection channel 170 is branched from the first sample outlet channel 150 at a second joint JB of the first sample outlet channel 150 and includes an ejection hole 171, wherein the first joint JA is positioned between the inlet end 152 and the second joint JB of the first sample outlet channel 150. In the embodiment, the first sample outlet channel 150 may be divided into an entrance section 150A extending from the inlet end 152 to the first joint JA, a buffer section 150B extending from the first joint JA to the second joint JB and a drain section 150C extending from the second joint JB to the distal end 154. In some embodiments, a channel diameter of the buffer section 150B may be gradually reduced from the first joint JA to the second joint JB.

The microfluidic device 10 further includes a processor PR and a guiding fluid source BS. The guiding fluid source BS is configured to provide a guiding fluid to the two first guiding channels 122 and 124 via respective guiding fluid inlet holes 128. The processor PR may control the guiding fluid source BS to determine the flow flux of the guiding fluid injected to the two first guiding channels 122 and 124. According to the present embodiment, an adjustment hole 126 passing through the outer surface of the microfluidic chip 100 may be further formed in the path of the first guiding channel 122, and an adjustment tube BAT connected to the guiding fluid source BS may be inserted into the adjustment hole 126. In this way, the processor PR may also control the guiding fluid source BS to additionally provide the guiding fluid through the adjustment tube BAT. In addition, a switch SW1 is attached to the adjustment tube BAT to control the fluid flow in the adjustment tube BAT. According to the present embodiment, each of the first sample inlet channel 110 and the two first guiding channels 122 and 124 allows a fluid from the exterior to enter the microfluidic chip 100 and may further include a filter section FS. The filter sections FS may each include a plurality of filtering slits for filtering unwanted particles, bubbles, or impurities in the entering fluid. Some exemplary embodiments of the structure of the filter sections FS have been disclosed in U.S. Patent Publication No. US20180326419A1, and the entire disclosure thereof is herein incorporated by reference.

The microfluidic device 10 further includes a pouring element PE connecting to an end of the pouring channel 160. In some embodiments, the pouring element PE may include a pouring tube PT connected to a guiding fluid source/tank (not shown) and a valve 161 attached to the pouring tube PT, wherein the pouring tube PT may be inserted to the hole formed at the end of the pouring channel 160. The processor PR may control the pouring element PE to pour guiding fluid into the pouring channel 160 by controlling the valve 161 to open. Specifically, when the valve 161 is opened, the guiding fluid is allowed to enter the pouring channel 160 from the guiding fluid source/tank through the pouring tube PT. In some embodiments, the pouring element PE may further include a pump capable of pushing the guiding fluid entering the pouring channel 160 to increase the flow rate of the guiding fluid in the pouring channel 160. In some embodiments, the pouring element PE and the first sorting assembly 120 may share the same guiding fluid source BS, but in alternative embodiments, the pouring element PE and the first sorting assembly 120 may connect to different guiding fluid sources.

The microfluidic device 10 also includes a flow adjustment element such as a valve 151 connected to the distal end 154 of the first sample outlet channel 150. The processor PR may control the valve 151 to open or close. According to the present embodiment, an intrinsic flow resistance to the fluid flow in the drain section 150C of the first sample outlet channel 150 is lower than an intrinsic flow resistance to the fluid flow in the collection channel 170. Once the processor PR controls the valve 151 to open, the drain section 150C of the first sample outlet channel 150 is a free channel without being clogged, thus the fluid flow arriving the second joint JB tends to enter the drain section 150C rather than the collection channel 170 due to the greater intrinsic flow resistance in the collection channel 170. To the contrary, when the processor PR controls the valve 151 to close, the flow resistance to the fluid flow in the drain section 150C increases and the fluid flow arriving the second joint JB tends to enter the collection channel 170. In some embodiments, the collection channel 170 may have a smaller channel diameter than the drain section 150C to increase the intrinsic flow resistance.

In general, the internal volume of the first sample outlet channel 150 between the first joint JA and the second joint JB (i.e. the buffer section 150B) is 0.01 µL-1000 µL. The internal volume of the first sample outlet channel 150 between the second joint JB and the distal end 154 (i.e. the drain section 150C) is 0.01 µL-1000 µL. The internal volume of the collection channel 170 is 0.01 µL-1000 µL. However, the disclosure is not limited thereto.

In some embodiments, the fluidic sample to be processed by the microfluidic device 10 may be a whole blood sample. The whole blood sample is injected into the first sample inlet channel 110 via the fluidic sample inlet hole 112. In some embodiments, the whole blood sample may be treated by mixing with a known reagent so as to dye target cells (particles) in the whole blood sample to be fluorescent. Thereafter, the treated whole blood sample is injected into the first sample inlet channel 110. Therefore, the fluorescent dyed target cells in the whole blood sample can be detected by an optical determination technique. For example, a linear light beam may be used to irradiate the whole blood sample travelling in the first sample inlet channel 110. Once the fluorescent dyed target cells in the whole blood sample pass through the linear light beam, the linear light beam may be absorbed or transferred to another wavelength, which allows the detection of the fluorescent dyed target cells. In the embodiment, the detection of the fluorescent dyed target cells may be performed at a section of the first sample inlet channel 110 adjacent to the first sorting chamber 130. However, the disclosure is not limited thereto.

Please refer to FIG. 1. When no fluorescent dyed target cell is detected in the fluidic sample travelling in the first sample inlet channel 110, the processor PR may control the flow flux of the guiding fluid in the first guiding channel 124 to be higher than the flow flux of the guiding fluid in the first guiding channel 122. In this way, the fluidic sample is guided into the first waste channel 140 and then drained from the waste outlet hole 142. In general, the valve 151 remains open and the valve 161 remains close. Some of the fluidic sample and the guiding fluid might stream into the first sample outlet channel 150 and be drained away from the distal end 154 of the first sample outlet channel 150. Namely, the distal end 154 of the first sample outlet channel 150 may not be clogged when no fluorescent dyed target cell is detected in the fluidic sample.

When at least one fluorescent dyed target cell is detected in the fluidic sample streaming in the first sample inlet channel 110, the processor PR may control the flow flux of the guiding fluid in the first guiding channel 124 to be lower than the flow flux of the guiding fluid in the first guiding channel 122. In one embodiment, the processor PR directly controls the guiding fluid source BS to provide guiding fluid with a higher flow flux to the first guiding channel 122 and provide guiding fluid with a lower flow flux to the first guiding channel 124. In another embodiment, the processor PR controls the switch SW1 to open so as to provide additional guiding fluid to the first guiding channel 122 such that the flow flux of the guiding fluid in the first guiding channel 124 can be lower than the flow flux of the guiding fluid in the first guiding channel 122. Therefore, the fluidic sample having the detected fluorescent dyed target cell TC is guided into the first sample outlet channel 150.

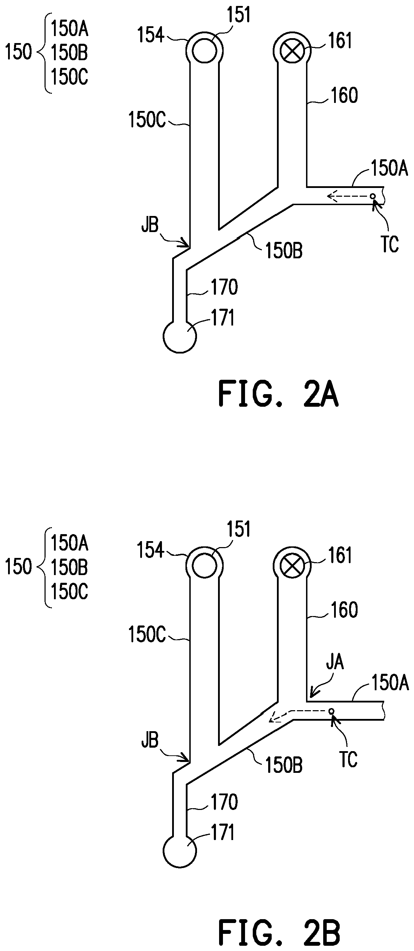

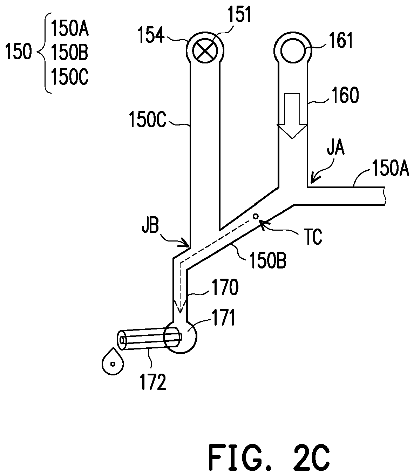

FIGS. 2A-2C shows how the dispensation of a detected fluorescent dyed target cell TC is achieved. In the present embodiment, as shown in FIG. 2A, when the switch SW1 is opened, the valve 151 is opened and the valve 161 is closed under the control of the processor PR, the fluidic sample having the detected fluorescent dyed target cell TC is allowed to move forward along the entrance section 150A, and then enter the buffer section 150B. In some embodiments, the position of the detected fluorescent dyed target cell TC in the fluidic sample may be determined based on the flow rate of the fluidic sample. Once the fluorescent dyed target cell TC enters the buffer section 150B as shown in FIG. 2B, the processor PR closes the valve 151 to increase the flow resistance in the drain section 150C and opens the valve 161 to pour guiding fluid into the pouring channel 160 as shown in FIG. 2C. Since the valve 151 is closed and the flow resistance in the drain section 150C is increased, the poured guiding fluid tends to stream towards the ejection hole 171 rather than the distal end 154 and hence pushes the detected fluorescent dyed target cell TC into the collection channel 170. In other words, the detected fluorescent dyed target cell TC eventually enters the collection channel 170 from the buffer section 150B and streams towards the ejection hole 171 under the push of the poured guiding fluid as shown in FIG. 2C. The fluorescent dyed target cell TC is then ejected from the ejection hole 171. Meanwhile, the valve 151 may be reopened, and the valve 161 may be closed again.

In some embodiments, a detection of the present of the fluorescent dyed target cell TC may be performed at a first predetermined location between the inlet end 152 and the first joint JA (i.e. the entrance section 150A) in the first sample outlet channel 150. According to one embodiment of the present invention, if a fluorescent dyed target cell TC is detected in the sorting chamber, the switch SW1 may be opened for a very short time (i.e. 7 ms) to prevent another fluorescent dyed target cell TC from entering the first sample outlet channel 150. Once the fluorescent dyed target cell TC reaches the first predetermined location, the switch SW1 may be closed. Thus, it ensures that only a single fluorescent dyed target cell TC exists in the first sample outlet channel 150.

The processor PR may close the valve 151 and open the valve 161 after a time interval after the switch SW1 is opened so as to guide the detected fluorescent dyed target cell TC to travel towards the collection channel 170 and consequently enable the detected fluorescent dyed target cell TC to be ejected through the ejection hole 171. However, the disclosure is not limited thereto. In one embodiment of the present invention, a detection of the present of the fluorescent dyed target cell TC may be performed at a second predetermined location between the first joint JA and the second joint JB (i.e. the buffer section 150B). When the fluorescent dyed target cell TC reaches the second predetermined location, the process may close the valve 151 and open the valve 161 to flush the fluorescent dyed target cell TC to the collection channel 170. The flushing will last until the fluorescent dyed target cell TC ejected by the droplet ejection device 172. The time interval can be determined by the distance between the first predetermined location and second predetermined location, and the flow flux of the fluidic sample in the first sample outlet channel.

According to some embodiments, the droplet ejection device 172 may be a nozzle inserted into the ejection hole 171, as shown in FIG. 2C. The nozzle is used to dispense a single droplet containing the fluorescent dyed target cell TC arriving the ejection hole 171. However, the disclosure is not limited thereto. For example, in another embodiment, a needle, a tube, or the like may be inserted into the ejection hole 171 to take out the fluorescent dyed target cell TC arriving the ejection hole 171.

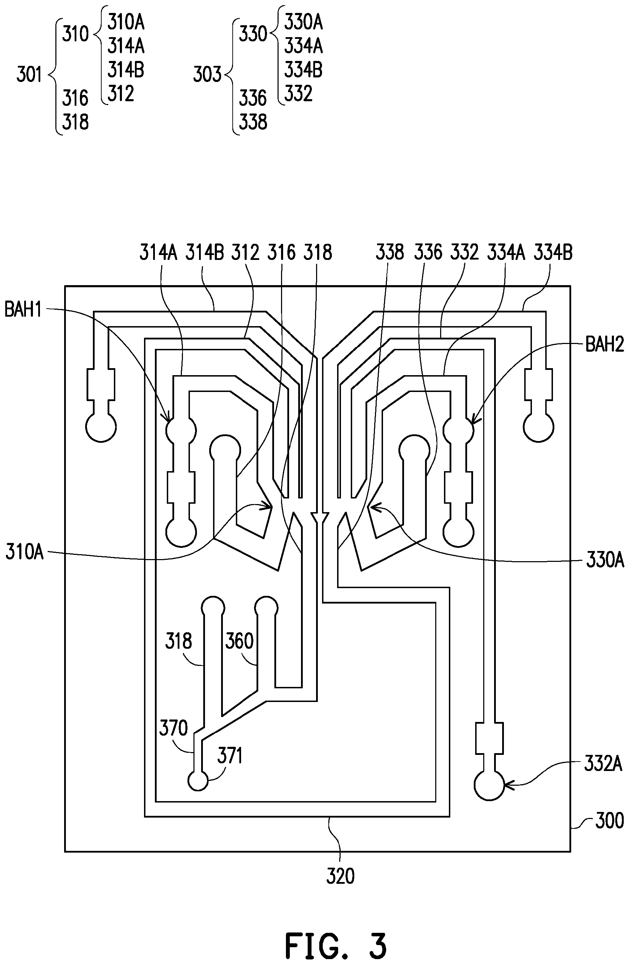

Refer to FIG. 3, which depicts a microfluidic chip according to an embodiment of the present application. The microfluidic chip 300 includes a first sorting system 301, a second sorting system 303 and a connection channel 320. The first sorting system 301 includes a first sorting assembly 310, a first sample outlet channel 318, a pouring channel 360, a collection channel 370 and a first waste channel 316. The first sorting assembly 310 includes a first sorting chamber 310A, a first sample inlet channel 312 and two first guiding channels 314A and 314B, wherein the first guiding channel 314A may include a first adjustment hole BAH1. The structure of the first sorting system 301 of FIG. 3 is similar with the structure of the microfluidic chip 100 of FIG. 1. The sorting and collecting mechanism of the first sorting system 301 is similar with the sorting and collecting mechanism of the microfluidic device 10. For the convenience of understanding, repeated and redundant description is hence omitted here.

In comparison to the microfluidic chip 100, the microfluidic chip 300 further includes the second sorting system 303 and the connection channel 320. The second sorting system 303 includes a second sorting assembly 330, a second sample outlet channel 338, and a second waste channel 336. The second sorting assembly 330 includes a second sorting chamber 330A, a second sample inlet channel 332 and two second guiding channels 334A and 334B, wherein the second guiding channel 334A may include a second adjustment hole BAH2. The connection channel 320 forms a fluidic communication between the second sample outlet channel 338 and the first sample inlet channel 312, as shown in FIG. 3.

The second sorting system 303 provides a preliminary sorting mechanism for the first sorting system 301. Specifically, a fluidic sample may be loaded into the second sample inlet channel 332 via the fluidic sample inlet hole 332A. A processor (not shown) is disposed to respectively control the flow flux of the guiding fluid in the two second guiding channels 334A and 334B. The fluidic sample is preliminarily sorted via the second sorting system 303. The sorting mechanism of the second sorting system 303 is similar to the sorting mechanism of the microfluidic device 10. For the convenience of understanding, repeated and redundant description is omitted.

After being sorted via the second sorting system 303, the fluidic sample sequentially streams in the second sample outlet channel 338, the connection channel 320 and the first sample inlet channel 312. Due to the preliminary sorting mechanism of the second sorting system 303, the concentration of the target cells in the fluidic sample (i.e. the purity of the fluidic sample) entering the first sample inlet channel 312 would be increased in comparison with the fluidic sample in the second sample inlet channel 332, which benefits the collection of the target cells via the ejection hole 371. In some embodiments of the present application, a droplet ejection device (not shown) is connected to the ejection hole 371 to dispense a single droplet containing one target cell arriving the ejection hole 371.

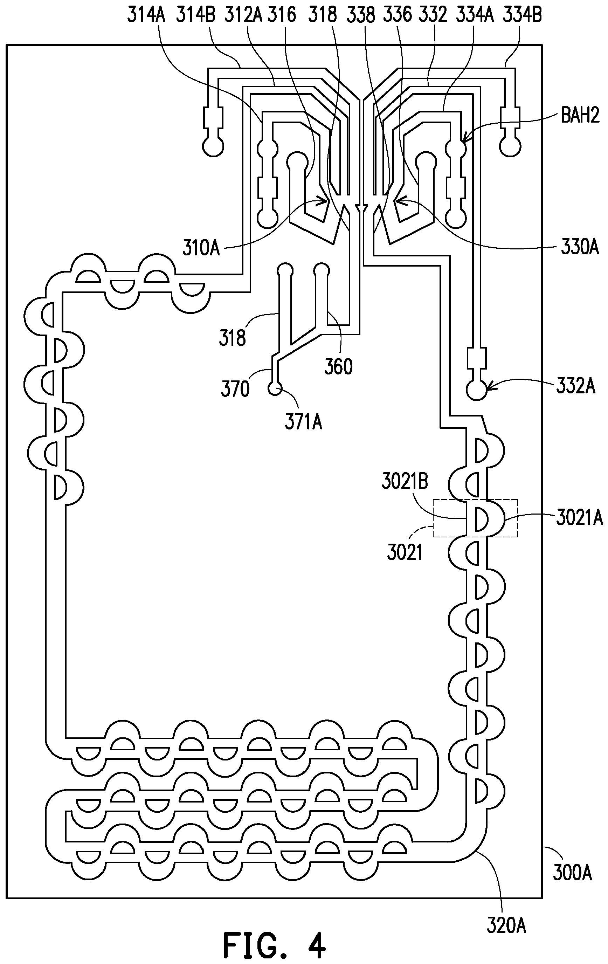

Refer to FIG. 4, which depicts a microfluidic chip according to an embodiment of the present application. In comparison to the microfluidic chip 300 of FIG. 3, the connection channel 320A of the microfluidic chip 300A of FIG. 4 further includes a plurality of longitudinal-particle-separation sections 3021 serially disposed along the extending direction of the connection channel 320A. Each of the longitudinal-particle-separation sections 3021 includes a winding portion 3021A and a shortcut portion 3021B, wherein the winding portion 3021A and the shortcut portion 3021B are connected in parallel between two joints at opposite terminals of each of the longitudinal-particle-separation sections 3021, and the path length of the winding portion 3021A is greater than the path length of the shortcut portion 3021B. In the present embodiment, the fluidic sample streams along the winding portion 3021A and the shortcut portion 3021B in different velocities, so as to further separate the target cells in the fluidic sample away from each other. In this way, the fluidic sample streaming in the first sample inlet channel 312A of the microfluidic chip 300A has target cells farther away from each other in comparison to the target cells of the fluidic sample streaming in the first sample inlet channel 312 of the microfluidic chip 300, which helps the operator or the user collect a single target cell among the target cells form the ejection hole 371A.

## Claims

1. A microfluidic device (10), comprising:
a microfluidic chip (100, 300, 300A), comprising:
a first sorting assembly (120, 310), including a first sorting chamber (130, 310A), a first sample inlet channel (110, 312, 312A), and two first guiding channels (122 124, 314A, 314B), wherein the first sample inlet channel (110, 312, 312A) and the two first guiding channels (122, 124, 314A, 314B) are converged at a first side (131) of the first sorting chamber (130, 310A), and the first sample inlet channel (110, 312, 312A) is positioned between the two first guiding channels (122, 124 314A, 314B);
a first sample outlet channel (150, 318), having an inlet end (152) reaching a second side (132) of the first sorting chamber (130, 310A) and a distal end (154) being distant from the inlet end (152);
a pouring channel (160, 360), branched from the first sample outlet channel (150, 318) at a first joint (JA) of the first sample outlet channel (150, 318);
a collection channel (170, 370), branched from the first sample outlet channel (150 318) at a second joint (JB) of the first sample outlet channel (150, 318) and including an ejection hole (171, 371, 371A) connected to a droplet ejection device (172) to dispense a single droplet; and
a first waste channel (140, 316), the first sorting chamber (130, 310A) bifurcating into the first waste channel (140, 316) and the first sample outlet channel (150, 318) at the second side (132),
wherein the first sample outlet channel (150, 318) consists of an entrance section (150A) positioned between the inlet end (152) of the first sample outlet channel (150, 318) and the first joint (JA), a buffer section (150B) positioned between the first joint (JA) and the second joint (JB), and a drain section (150C) positioned between the second joint (JB) and the distal end (154) of the first sample outlet channel (150, 318);
a pouring element (PE), connected to the pouring channel (160, 360);
a flow adjustment element, connected to the distal end (154) of the first sample outlet channel (150, 318), when actuated, a flow resistance to the fluid flow in the drain section (150C) being lower than a flow resistance to the fluid flow in the collection channel (170, 370); and
a processor (PR), configured to control the pouring element (PE) to pour a guiding fluid into the pouring channel (160, 360) entering the buffer section (150B) of the first sample outlet channel (150, 318) and control the flow adjustment element to adjust a flow resistance of the drain section (150C) of the first sample outlet channel (150, 318).

2. The microfluidic device (10) of claim 1, wherein a channel diameter of the collection channel (170, 370) is smaller than a channel diameter of the drain section (150C) of the first sample outlet channel (150, 318).

3. The microfluidic device (10) of claim 1 or 2, wherein the pouring element (PE) comprises a pouring tube (PT) connected to an end of the pouring channel (160, 360) and a valve (161) attached to the pouring tube (PT), wherein the processor (PR) controls the valve (161) to open and close.

4. The microfluidic device (10) of claim 1, wherein the flow adjustment element comprises a valve (151), and the processor (PR) controls the valve (151) to open and close.

5. The microfluidic device (10) of any of claims 1 to 4, wherein the microfluidic chip (100, 300, 300A) further comprises:
a second sorting assembly (330), including a second sorting chamber (330A), a second sample inlet channel (332), and two second guiding channels (334A, 334B), wherein the second sample inlet channel (332) and the two second guiding channels (334A, 334B) are converged at a first side (131) of the second sorting chamber (330A), and the second sample inlet channel (332) is positioned between the two second guiding channels (334A, 334B);
a second sample outlet channel (338);
a connection channel (320, 320A), forming a fluidic communication between the second sample outlet channel (338) and the first sample inlet channel (110, 312, 312A); and
a second waste channel (336), the second sorting chamber (330A) bifurcating into the second waste channel (336) and the second sample outlet channel (338) at the second side (132).

6. The microfluidic device (10) of claim 5, wherein the connection channel (320, 320A) comprises a plurality of longitudinal-particle-separation sections (3021) serially connected to each other along an extending direction of the connection channel (320, 320A), each of the longitudinal-particle-separation sections (3021) comprises at least one winding portion (3021A) and at least one shortcut portion (3021B), wherein the winding portion (3021A) and the shortcut portion (3021B) are connected in parallel between two joints at opposite terminals of the each of the longitudinal-particle-separation sections (3021), and the path length of the winding portion (3021A) is greater than the path length of the shortcut portion (3021B).

7. The microfluidic device (10) of claim 1, wherein the droplet ejection device (172) is a nozzle inserted into the ejection hole (171, 371, 371A).

8. The microfluidic device (10) of claim 1, wherein a channel diameter of the buffer section (150B) of the first sample outlet channel (150, 318) is gradually reduced from the first joint (JA) to the second joint (JB).

9. A microfluidic chip (100, 300, 300A), comprising:
a first sorting assembly (120, 310), including a first sorting chamber (130, 310A), a first sample inlet channel (110, 312, 312A), and two first guiding channels (122, 124, 314A, 314B), wherein the first sample inlet channel (110, 312, 312A) and the two first guiding channels (122, 124, 314A, 314B) are converged at a first side (131) of the first sorting chamber (130 310A), and the first sample inlet channel (110, 312, 312A) is positioned between the two first guiding channels (122, 124, 314A, 314B);
a first sample outlet channel (150, 318), extending to reach a second side (132) of the first sorting chamber (130, 310A) at an inlet end (152) of the first sample outlet channel (150, 318);
a pouring channel (160, 360), branched from the first sample outlet channel (150, 318) at a first joint (JA) of the first sample outlet channel (150, 318);
a collection channel (170, 370), branched from the first sample outlet channel (150, 318) at a second joint (JB) of the first sample outlet channel (150, 318) and including an ejection hole (171, 371, 371A) connected to a droplet ejection device (172) to dispense a single droplet; and
a first waste channel (140, 316), the first sorting chamber (130, 310A) bifurcating into the first waste channel (140, 316) and the first sample outlet channel (150, 318) at the second side (132);
wherein the first sample outlet channel (150, 318) consists of an entrance section (150A) positioned between the inlet end (152) of the first sample outlet channel (150, 318) and the first joint (JA), a buffer section (150B) positioned between the first joint (JA) and the second joint (JB), and a drain section (150C) positioned between the second joint (JB) and the distal end (154) of the first sample outlet channel (150, 318);
wherein a flow resistance to the fluid flow in the drain section (150C) is lower than a flow resistance to the fluid flow in the collection channel (170, 370).

10. The microfluidic chip (100, 300, 300A) of claim 9, wherein a channel diameter of the collection channel (170, 370) is smaller than a channel diameter of the drain section (150C) of the first sample outlet channel (150, 318).

11. The microfluidic chip (100, 300, 300A) of claim 9 or 10, further comprises:
a second sorting assembly (330), including a second sorting chamber (330A), a second sample inlet channel (332), and two second guiding channels (334A, 334B), wherein the second sample inlet channel (332) and the two second guiding channels (334A, 334B) are converged at a first side (131) of the second sorting chamber (330A), and the second sample inlet channel (332) is positioned between the two second guiding channels (334A, 334B);
a second sample outlet channel (338);
a connection channel (320, 320A), forming a fluidic communication between the second sample outlet channel (338) and the first sample inlet channel (110, 312, 312A); and
a second waste channel (336), the second sorting chamber (330A) bifurcating into the second waste channel (336) and the second sample outlet channel (338) at the second side (132) of the second sorting chamber (330A).

12. The microfluidic chip (100, 300, 300A) of claim 11, wherein the connection channel (320, 320A) comprises a plurality of longitudinal-particle-separation sections (3021) serially connected to each other along an extending direction of the connection channel (320, 320A), each of the longitudinal-particle-separation sections (3021) comprises at least one winding portion (3021A) and at least one shortcut portion (3021B), wherein the winding portion (3021A) and the shortcut portion (3021B) are connected in parallel between two joints at opposite terminals of the each of the longitudinal-particle-separation sections (3021), and the path length of the winding portion (3021A) is greater than the path length of the shortcut portion (3021B).

13. The microfluidic chip (100, 300, 300A) of claim 9, wherein a channel diameter of the buffer section (150B) of the first sample outlet channel (150, 318) is gradually reduced from the first joint (JA) to the second joint (JB).
